# EUROPEAN PATENT SPECIFICATION

(11) **EP 2 491 890 B1**
(45) Date of publication and mention of the grant of the patent: **29.01.2014**
(21) Application number: 12157112.9
(22) Date of filing: 27.02.2012
(51) Int. Cl.: A61F 2/00, A61F 2/14

(54) **System and method for preparing a lenticular or corneal implant**
System und Verfahren zur Herstellung eines Linsen- oder Hornhautimplantats
Système et procédé pour préparer un implant cornéen ou lenticulaire

(30) Priority: 28.02.2011 GB 201103384; 20.12.2011 US 201113374292
(43) Date of publication of application: 29.08.2012
(73) Proprietor: Weston, Philip Douglas, Grantley, North Yorkshire HG4 3PJ (GB); Tan, Donald, Singapore 267054 (SG); Mehta, Jod, Singapore 138686 (SG)
(72) Inventor: Weston, Philip Douglas, Grantley, North Yorkshire HG4 3PJ (GB); Tan, Donald, Singapore 267054 (SG); Mehta, Jod, Singapore 138686 (SG)
(74) Representative: Vaughan, Christopher Tammo

(56) References cited:
- EP-A1- 2 002 803
- US-A- 5 616 148
- US-A- 6 143 001
- US-A1- 2006 200 167
- US-A1- 2010 211 051
- US-B1- 6 605 093
- WEI-BOON KHOR ET AL: "Descemet Stripping Automated Endothelial Keratoplasty With a Graft Insertion Device: Surgical Technique and Early Clinical Results", AMERICAN JOURNAL OF OPHTHALMOLOGY, vol. 151, no. 2, 1 February 2011 (2011-02-01), pages 223-232.E2, XP55030016, ISSN: 0002-9394, DOI: 10.1016/j.ajo.2010.08.027

## Description

### BACKGROUND

Embodiments of this invention relate to a system and method for handling and inserting a corneal implant (also referred to as a donor cornea or lenticule) into the eye of a recipient without inducing significant endothelial damage.

A paradigm shift in the approach to corneal transplantation is occurring, with new forms of anterior and posterior lamellar keratoplasty now enabling targeted replacement of only diseased layers of the cornea. These forms of lamellar corneal surgery are gradually replacing conventional full thickness penetrating keratoplasty (Tan DT, Mehta JS: "Future Directions in Lamellar Corneal Transplantation"; Cornea; October 2007; Volume 26; pp S21-S28).

Descemet's stripping automated endothelial keratoplasty (DSAEK) is a form of small incision and essentially sutureless surgery which represents the latest innovation in a series of posterior lamellar keratoplasty procedures that are now synonymous with the term "endothelial keratoplasty". The DSAEK procedure involves stripping of diseased Descemet's membrane and endothelial cells through a small corneal incision, and replacement with a posterior lamellar donor corneal lenticule prepared with the use of the Automated Lamellar Therapeutic Keratoplasty (ALTK) unit (Price MO, Price FW Jr.: "Descemet's stripping with endothelial keratoplasty: comparative outcomes with microkeratome-dissected and manually dissected donor tissue"; Ophthalmology; 2006 Nov; 113(11):1936-42).

With the adoption of any new surgical technique there is an inevitable learning curve for the surgeon and an accompanying evolution in techniques (see, for example: Price FW, Price MO: "Descemet's stripping with endothelial keratoplasty in 200 eyes: Early challenges and techniques to enhance donor adherence"; J Cataract Refract Surg. 2006; 32(3):411-8; Melles GR, Lander F, Beekhuis WH, Remeijer L, Binder PS: "Posterior lamellar keratoplasty for a case of pseudophakic bullous keratopathy"; Am J Ophthalmol. 1999 Mar; 127(3):340-1; Melles GR, Lander F, Nieuwendaal C: "Sutureless, posterior lamellar keratoplasty: a case report of a modified technique"; Cornea; 2002 Apr; 21(3):325-7; Melles GR, Wijdh RHJ, Nieuwendaal CP: "A technique to excise the Descemet membrane from a recipient cornea (descemetorhexis)"; Cornea; 2004 Apr; 23(3):286-8; Terry MA, Ousley PJ: "Replacing the endothelium without corneal surface incisions or sutures: the first United States clinical series using the deep lamellar endothelial keratoplasty procedure"; Ophthalmology; 2003 Apr; 110:755-64; discussion 764). US6,605,093 discloses a device and method for use with an ophthalmologic inserter apparatus.

One of the most challenging aspects of this procedure is the insertion of the donor posterior lenticule into the anterior chamber (AC) through a small incision, without inducing significant endothelial damage. The current widely performed technique requires insertion of the donor lenticule through a small 5mm corneal or scleral incision by folding the lenticule and gripping the folded tissue with non-compressing forceps i.e. 'taco insertion'. This traumatic handling of the donor has been criticized because of its propensity for damaging endothelial cells, with primary graft failure rates due to intraoperative endothelial cell loss and damage ranging from 6% to 45% in the current literature with this folding technique (Mearza AA, Qureshi MA, Rostron CK: "Experience and 12-month results of Descemet-stripping endothelial keratoplasty (DSEK) with a small-incision technique"; Cornea 2007 Apr; 26(3):279-283). Damage to endothelial cells may occur as a consequence of mechanical folding of the donor, compression with holding forceps, and may also occur during intraocular manipulations to unfold the donor within the AC without the presence of an ophthalmic visco-surgical device (OVD). More recently, laboratory models of DSAEK have shown that folding of the donor lenticule for insertion into the AC and intraocular manipulation to unfold the donor is the stage most associated with significant endothelial cell loss (Lee WB, Sy HM, Holley GP, Edelhauser HF: "Descemet's Stripping Automated Endothelial Keratoplasty (DSAEK): Intra-Operative Effects on the Donor Corneal Endothelium"; IOVS supplement; 2007; abstract 1131). The endothelial damage is worse in the presence of associated anterior chamber shallowing.

Our own extensive in-vitro work has confirmed that significant endothelial damage occurs with the conventional folding technique, despite the use of commercially available 'non-compression' forceps (Goosey forceps, model no. 19090, Moria, Antony, France). Damage primarily occurring as a consequence of direct contact of folded endothelial surfaces where the folding forceps are applied, as well as along the folding crease (Mehta JS, Por YM, Beuerman RW, Tan DT: "Glide Insertion Technique of Donor Cornea Lenticule during Descemet's Stripping Automated Endothelial Keratoplasty"; J Cat Refract Surg; in press). Our recent studies show that the mean endothelial cell loss is 39% with this technique, which is now described:

A 1 mm paracentesis is first made in the peripheral cornea opposite a 5mm temporal scleral tunnel wound (for insertion of intraocular forceps). A standard, commercially available anterior chamber intraocular lens (IOL) Sheet's glide is trimmed to 4mm in width along approximately half to 2/3 of its length. Using Kelman Macpherson forceps, the glide is inserted into the AC through the scleral tunnel, with the right hand, whilst a balanced saline solution (BSS) infusion is maintained on. The donor (both the anterior and posterior lamellae) is transferred to a Paton's spatula. A dispersive OVD is liberally applied over the endothelial surface particularly the peripheral circumference of the donor. Carefully gripping the posterior donor lamellar with Kawai intraocular capsulorhexis forceps (Asico) on the stromal side, the anterior cap is slid away from the spatula, ensuring that the posterior donor lamella stays on the spatula. OVD is placed on the anterior surface of the glide, and the Paton spatula with the posterior lenticule is carefully everted, corneal endothelial surface down, onto the OVD-covered portion of the glide. Holding the glide with the right hand with Kelman Macpherson forceps at its most posterior part, the left hand, passes the Kawai forceps through the paracentesis, across the AC and over the sheets glide, and is passed out through the scleral incision. The Kawai forceps is rotated, so that the forceps teeth are now obliquely or vertically aligned, and can be used to grasp the leading edge of the donor lamella, on the upper stromal surface. Once the forceps grasped the donor edge, the donor is rapidly pulled through the scleral incision in one steady, smooth motion until the donor is fully in the AC. At the same time, the glide was retracted out of the eye.

We have performed this technique in 24 cases of DSAEK surgery, with only one primary graft failure occurring (4.2%). This contrasts with our previous 20 cases using the folding technique which had primary graft failure rate of 25% (5 eyes). Our scanning electron microscope (SEM) studies confirm that significant reduction in endothelial loss occurs with this technique, with a mean cell loss of 9%, mostly occurring at the peripheral rim, which may be due to contact of the donor edges with the plastic sheets glide, despite the use of OVD, and some damage must still occur when the donor is dragged through the lips of the wound, as the donor endothelial surface is still potentially in contact with the inferior lip of the scleral wound. We have not encountered any cases of donor dislocation with this technique, although we have now seen one case of partial Descemet's detachment. Our only primary graft failure occurred during our first case using this technique and can be attributed to the use of an excessively thick donor lenticule (400pm) which resulted in Descemet's detachment.

Recently, a new technique called Descemet's Membrane Endothelial Keratoplasty (DMEK) has been developed. In this endothelial keratoplasty technique, an isolated an isolated Descemet's membrane is transplanted. This technique is even more difficult than DSAEK surgery, since an isolated Descemet's membrane is even thinner and more fragile than one which is supported by one or more layers of endothelial cells.

A previous system and method developed by the present Applicants in order to facilitate DSEAK surgery is described in WO2009/050511, and further in

US2010/0211051. This system for donor cornea implantation includes a preparation base having a well for receiving a donor cornea, a cartridge disengageably mounted on the base adjacent the well, and a handle for disengageable attachment to a posterior end portion of the cartridge. In drawing the donor cornea from the well into and through a bore or chamber of the cartridge, from the posterior end, the donor cornea is caused to assume a double coil configuration. After attachment of the handle, removal of the assembly from the preparation base, and insertion of a blade and adjacent body portions of the cartridge through an incision in the recipient's cornea, the coiled donor cornea is pulled from the cartridge chamber, through its forward end, to uncoil automatically within the anterior chamber of the recipient's eye. While effective, it is believed that there is still room for improvement.

### BRIEF SUMMARY OF THE DISCLOSURE

Embodiments of the invention may seek to provide an apparatus and method for inserting an endothelial corneal implant (which may be a donor implant harvested from a cadaver, or alternatively an artificial endothelial implant) into the eye of a recipient without inducing significant endothelial damage. The endothelial implant may comprise an isolated Descemet's membrane, or a Descemet's membrane supported on one or several layers of endothelial cells. The endothelial implant may also be referred to as a donor cornea or lenticule.

Further embodiments of the invention may seek to provide such an apparatus and method wherein and whereby the donor cornea is temporarily deformed for effective insertion, while providing protection against significant endothelial damage.

Viewed from a first aspect, the present invention provides a system for use in preparing a lenticular or corneal implant, in particular but not exclusively an endothelial corneal implant, for delivery to an operating site and insertion into a recipient's eye, the system comprising:
a cartridge comprising a generally tubular portion including a sidewall defining a longitudinal bore, of curvilinear cross section, having open forward and rearward ends;
a preparation base comprised of a lower portion and an upper portion, said upper portion having a recess and having cartridge-receiving structure for removably engaging said cartridge in position with said open rearward end of said longitudinal bore thereof proximate one side of said recess, and for allowing facile removal of said cartridge from said base; and
an insert member having structure for removably engaging said insert member in said recess, said insert member having a portion defining a platform for receiving the implant, and a portion adjacent the platform defining funnel structure having a wider end facing the platform, and a narrower end facing the open rearward end of said cartridge when the system is assembled; and
wherein the insert member has a saddle-shaped configuration, adapted to straddle and snugly engage the recess in the upper portion of the preparation base.

The cartridge may comprise attaching structure adjacent a rearward end of said tubular portion for disengageable attachment to a handle.

The system may further comprise a handle having a gripping portion and means adjacent one end for disengageable attachment to said disengageable attachment structure of said cartridge, said handle, when attached, enabling facile manipulation of said cartridge.

The upper portion of said base may be constructed to accommodate said handle when it is assembled with said engaged cartridge.

The attachment means of said handle may engage said attachment structure of said cartridge in only a single orientation of relative rotation about a longitudinal axis.

The attachment means of said handle engages attachment structure of said cartridge in a snap-fit relationship.

The handle may have closure structure at said one end thereof, constructed to engage said tubular portion of said cartridge and to thereby produce a liquid-tight seal of said normally open rearward end of said bore of said tubular portion.

The gripping portion of the handle may have opposite sides, and indicia may be provided on at least one of said opposite sides of said gripping portion to distinguish it from the other side thereof.

At least one of said cartridge and said funnel structure may have structure for inducing inward curling of opposite lateral edges of the implant in the course of passing from said platform to said forward end of said bore of said cartridge.

The narrower end of the funnel structure of the insert member may define an aperture similar in cross-sectional area to the cross-section of the longitudinal bore or the cartridge.

The funnel structure may further include a first ridge element extending longitudinally along at least a portion of funnel structure proximate the narrower end thereof and projecting inwardly thereinto. The ridge element may serve to induce curling of lateral edges of the implant during longitudinal movement therealong from the wider end to the narrower end and into the longitudinal bore of the cartridge.

Alternatively or in addition, the longitudinal bore of the cartridge may include a second ridge element extending longitudinally along at least a portion of the bore and projecting inwardly thereinto from the sidewall and serving to induce curling of lateral edges of the implant during longitudinal movement therealong.

Where both first and second ridge elements are provided, these should be in general positional registration with each other when the insert member and the cartridge are assembled onto the preparation base, and of generally similar cross-sectional shape and/or size where ends of the first and second ridge elements face each other.

The insert member has a saddle-shaped configuration, adapted to straddle and snugly engage the recess in the upper portion of the preparation base. Viewed in an alternative manner, the insert member may have a bridge-type structure, with a spanning portion and two leg portions. The spanning portion bridges the preparation base, and the leg portions engage on either side of the preparation base. Outer parts of the leg portions may be provided with finger grips to facilitate placement and removal of the insert member on or from the preparation base.

The platform of said insert member may cooperate with platform structure defined on the upper portion of said preparation base so as to provide a generally dish-shaped receptacle for receiving the implant. In other embodiments, the platform of said insert member by itself defines a generally dish-shaped receptacle for receiving the implant. The system may include two or more insert members that define dish-shaped receptacles of differing depths or curvatures so as to accommodate different thicknesses of implant. For some surgical procedures, for example, it is desirable to use a very thin single lenticule comprising just endothelial tissue, whereas other procedures may require a thicker double lenticule comprising a full thickness graft of thickness 80 to 120µm.

The cartridge may have a blade portion extending forwardly from said sidewall beyond said forward open end of said bore. The blade portion may be suitable for insertion into an incision in a corneal surface of a recipient eye.

The cartridge will advantageously be integrally formed, as a single piece, and will desirably be moulded from a substantially transparent or translucent synthetic resinous material. The ridge element where provided will normally be formed with convexly curved lateral surfaces extending along its length and terminating in a common longitudinal apex. At the forward end of the tubular portion of the cartridge the sidewall will desirably be formed with a transaxial bevel that declines toward the blade portion, to facilitate physical access into the bore and insertion of the forwardmost part of the body portion into the recipient's cornea. The curvilinear cross section of the bore will normally be generally circular or generally elliptical, and the bore will generally be of uniform cross-section along at least a major portion of its length.

The structure for inducing bilateral curling of a donor cornea may comprise a ridge element extending longitudinally along the bore of the cartridge and/or in the funnel structure, as described. Additionally, there may be provided a pair of laterally spaced shoulder elements, having curl-inducing surfaces facing one another and disposed substantially at the intersection of the dish-shaped receptacle and the cartridge-receiving structure (taking the form, for example, of curved surfaces forming a U-shaped throat section). Normally, the cartridge-receiving structure of the preparation base will coact slideably with structure of the cartridge in such manner that the cartridge can be received in only a single orientation of rotation about its longitudinal bore, and usually the cartridge-receiving structure and the attaching structure will permit receipt of the cartridge by relative movement in only one direction, and disengagement by relative movement only in the opposite direction.

The lower portion of the preparation base may be flat so as to stand on a surface such as a worktop without wobbling. The lower portion of the preparation base may include a flange and/or finger grips so as to allow it to be held firmly on the worktop without slipping.

Viewed from another aspect, the present invention provides a method of preparing a lenticular or corneal implant, in particular but not exclusively an endothelial corneal implant, prior to surgery, using the system described above, comprising the steps:
(1) supporting the implant in position adjacent the open rearward end of said longitudinal bore of said tubular portion of said cartridge; and
(2) drawing said implant into said bore, to reside therein, by force applied from said open forward end of said bore.

Optionally, the method may further comprise the step of:
(3) attaching said handle to said cartridge using said means for disengageable attachment and said disengageable attachment structure of said cartridge.

The implant may be an endothelial corneal implant, in which case it is supported adjacent the open rearward end of the longitudinal bore with its endothelial side uppermost, and cause to adopt a double coil configuration within the longitudinal bore with the endothelial surface on the inside of the double coil, so as to provide a degree of protection from damage.

In use, an endothelial corneal implant is placed, endothelial surface facing upwardly, in position adjacent the open rearward end of the longitudinal bore of the tubular portion of the cartridge; the donor cornea is drawn into the bore, to reside therein, by force applied from the open forward end of the bore, for example by pulling the implant into the cartridge by way of forceps which have been introduced at the forward end of the bore and through the cartridge. As the implant is drawn through the funnel structure, opposed edges of the implant curl inwardly and the implant maintains a double-curl configuration within the bore of the cartridge. Once the implant is fully within the cartridge, the forceps are released, and the handle is attached to the cartridge using the means described.

During subsequent surgery, the cartridge may be positioned, using the attached handle, so that the blade portion enters an incision and provides a platform along which the implant may glide into the anterior chamber of a recipient eye. The implant is typically drawn through the forward open end of the cartridge bore into the anterior chamber of the recipient's eye using forceps.

During surgery, an opening (incision or paracentesis) is formed in the cornea of a recipient, at least at generally diametrical locations; followed by inserting the blade portion and the adjacent part of the body portion of the cartridge through one of the openings, inserting a gripping portion of an instrument through another opening in the cornea so as to grip an edge of the implant adjacent the open forward end of the cartridge bore; and pulling the implant into the anterior chamber of the recipient eye. The method may include the step of causing the implant to assume a double coil configuration, with no endothelial surface areas in mutual contact with one another, in the course of drawing the implant into the bore of the cartridge.

A cartridge for effecting coiling and insertion of a donor cornea implant, may comprise a generally tubular portion including a sidewall defining a longitudinal bore, of curvilinear cross section, having open forward and rearward ends; a blade portion extending forwardly from said sidewall beyond said forward open end of said bore; and structure adjacent a rearward end of said tubular portion for disengageable attachment to a handle; wherein the sidewall of the cartridge includes a portion that is tapered from the rearward end to the forward end, such that the longitudinal bore has a greater cross-section at the rearward end than the forward end.

In some embodiments, the cartridge may further comprise a ridge element extending longitudinally along at least a portion of said bore and projecting inwardly thereinto from said sidewall, said ridge element being constructed to induce inward curling of opposite lateral edges of a donor cornea during movement therealong.

In some embodiments, an outer surface of the sidewall may be provided with ridges, ribs, grooves or other structure to help to retain the cartridge in place when inserted through an incision into the anterior chamber of a recipient's eye.

By tapering the structure of the cartridge from its rearward end to its forward end, it is possible to provide a large enough opening at the rearward end to facilitate insertion in coiling of the donor cornea implant as described hereinabove, while allowing the forward end and the blade portion to be narrower than hitherto possible. When making an incision into the surface of an eye, for example into the anterior chamber, it is better for the incision to be made as small as possible. This is because the anterior chamber (and indeed the eye itself) has a tendency to invert or turn inside out due to internal pressure if too large an incision is made.

Moreover, by providing external ribs or grooves or ridges on at least a portion of the outer surface of the sidewall, it is possible to design the cartridge so that it tends to stay in place when inserted into the anterior chamber of an eye through a small incision and will tend to resist extrusion due to pressure from the inside of the anterior chamber. The ribs or grooved or ridges are preferably configured to as to be substantially parallel to the sides of the incision when the cartridge is inserted into the anterior chamber.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents.

### BRIEF DESCRIPTION OF THE SEVERAL VIEWS OF THE DRAWINGS

For a better understanding of the present invention and to show how it may be carried into effect, reference shall now be made by way of example to the accompanying drawings, in which:
Figure 1 is a top perspective view of an embodiment of the system of the invention in a first configuration;
Figure 2 is a top perspective view of an embodiment of the system of the invention in a second configuration;
Figure 3 is an enlarged perspective view of an insert member shown in Figure 1;
Figure 4 is a rear elevation of the insert member of Figure 3;
Figure 5 is a perspective view of the handle and cartridge of embodiments of the system of the invention, separated from one another and taken from the bottom (relative to the orientation in which corneal implant insertion is effected);
Figure 6 is a bottom view of the handle and cartridge in assembly with one another;
Figure 7 is a plan view of the assembly of Figure 6;
Figure 8 is a fragmentary perspective view of the forward portion of the preparation base, depicting the placement of an implant into a well formed in the top wall of the base;
Figure 9 is a fragmentary perspective view of the forward portion of the preparation base with the cartridge component engaged thereon;
Figure 10 is a view similar to Figure 9, showing an implant being drawn into the rear, or posterior, opening of the cartridge bore, or chamber;
Figure 11 is a perspective view of the cartridge of the system, drawn to an enlarged scale and containing an implant in a double coil configuration;
Figure 12 is a cross sectional view taken along line 28 - 28 in Figure 11, drawn to an enlarged scale and showing the donor cornea formed into a double coil configuration;
Figure 13 is a perspective view showing the forward portion of the cartridge inserted through a scleral tunnel of a cornea, and also showing forceps introduced through a nasal paracentesis gripping the stromal leading edge of the implant and pulling it into the anterior chamber;
Figure 14 is a perspective view similar to Figure 13, showing the substantially uncoiled implant being manipulated within the anterior chamber;
Figure 15 is another similar perspective view showing the implant released and the cartridge and forceps removed;
Figure 16 is a perspective view of an alternative cartridge having a tapered configuration; and
Figure 17 is an underplan view of the cartridge of Figure 16.

### DETAILED DESCRIPTION OF THE INVENTION

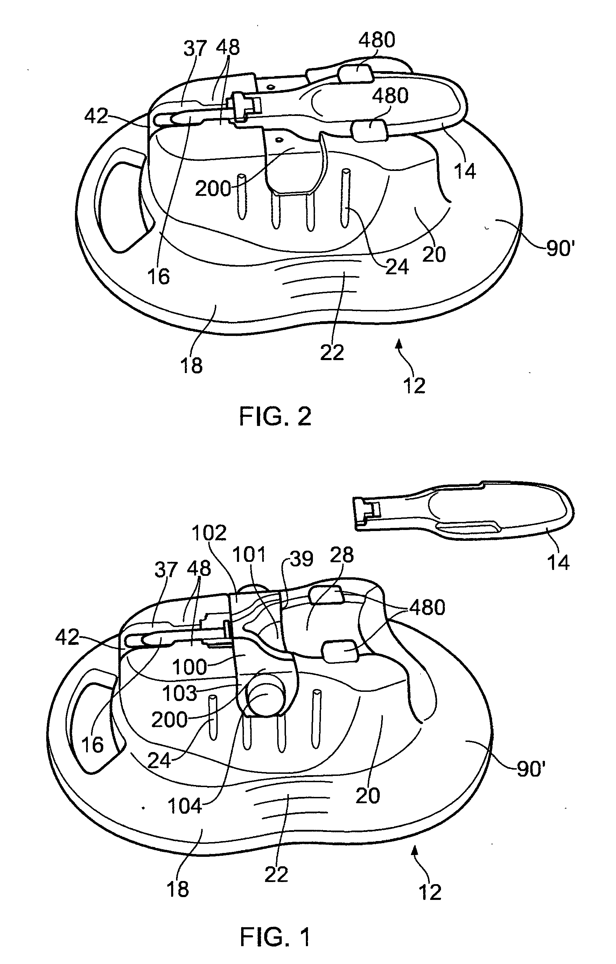

Figure 1 shows an embodiment of the system of the invention, which comprises a preparation base, a handle, and a cartridge, generally designating respectively by the numerals 12, 14, and 16. Each of these components will desirably be moulded from a suitable synthetic resinous (that is, plastic) material.

The preparation base 12 is symmetrical about a longitudinal centreline, and includes a bottom flange portion 18 and an upstanding pedestal portion 20, each of which is provided with gripping elements 22, 24, respectively. It will be noted that the flange portion 18 has the word CORONET, designated 90', moulded into it so as to the project above the adjacent surface; apart from its literal significance, the structure forming the word provides texture to further facilitate secure gripping of the base.

An implant-receiving recess or well 28, formed in the top wall of the pedestal portion 20 of the preparation base 12, and complemented by a platform 101 formed in a saddle- or bridge-shaped insert member 100, which will be described in more detail below. A relatively wide trough 39, of arcuate cross section, is also formed in the top wall of the pedestal portion 20 and extends rearwardly from the well 28.

A pair of lugs 48 extend laterally inwardly toward one another at the top of the pedestal portion 20, and define the upper margins of a passage 37 between the well 28 and a channel 42. The lugs 48 serve to engage with and releasably retain the cartridge 16 as shown. A further pair of lugs 480 on the top of the pedestal portion 20 serve to engage with and releasably retain the handle 14 when engaged with the cartridge 16 as shown best in Figure 2.

Figure 2 shows a recess 200 in the pedestal portion 20 of the preparation base 12, the recess 200 running from one side of the pedestal portion 20 to the other generally perpendicular to the channel 42. The recess 200 is configured releasably to receive the saddle- or bridge-shaped insert member 100 as shown best in Figure 1. The insert member 100 may clip into place or be held in place by a friction fit. The platform 101 of the insert member 100 complements the well 28 to define a platform for receiving an implant (not shown). The insert member 100 has a span portion 102 and a pair of legs 103. The legs 103 are provided with finger grips 104.

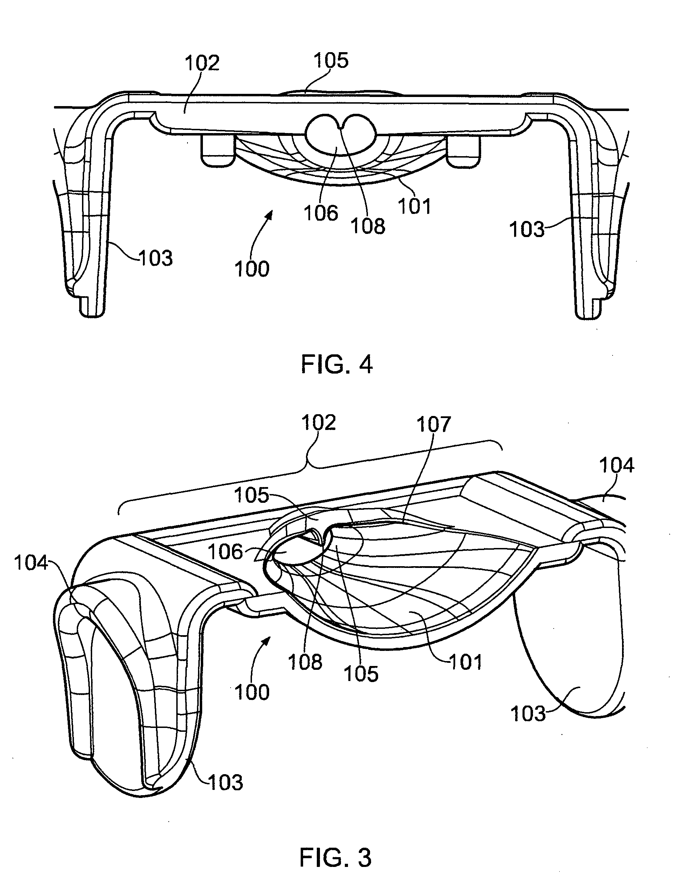

Figures 3 and 4 show the insert member 100 in more detail, with its span portion 102, two legs 103, finger grips 104 and platform 101. The platform 101 is dished, and together with an upper part of the span portion 102 defines a generally funnel shaped structure 105 with a narrower end or spout 106 and a wider end or mouth 107. In preferred embodiments, the interior of the funnel structure 105 incorporates a longitudinal ridge 108. The ridge 108, as best shown in Figure 4, together with a generally curvilinear inner surface profile of the funnel structure 105, results in the spout 106 of the funnel structure 105 having a pretzel-like cross-section, the purpose of which is to cause or encourage curling of a corneal implant in a desired manner as the implant is pulled through the spout 106.

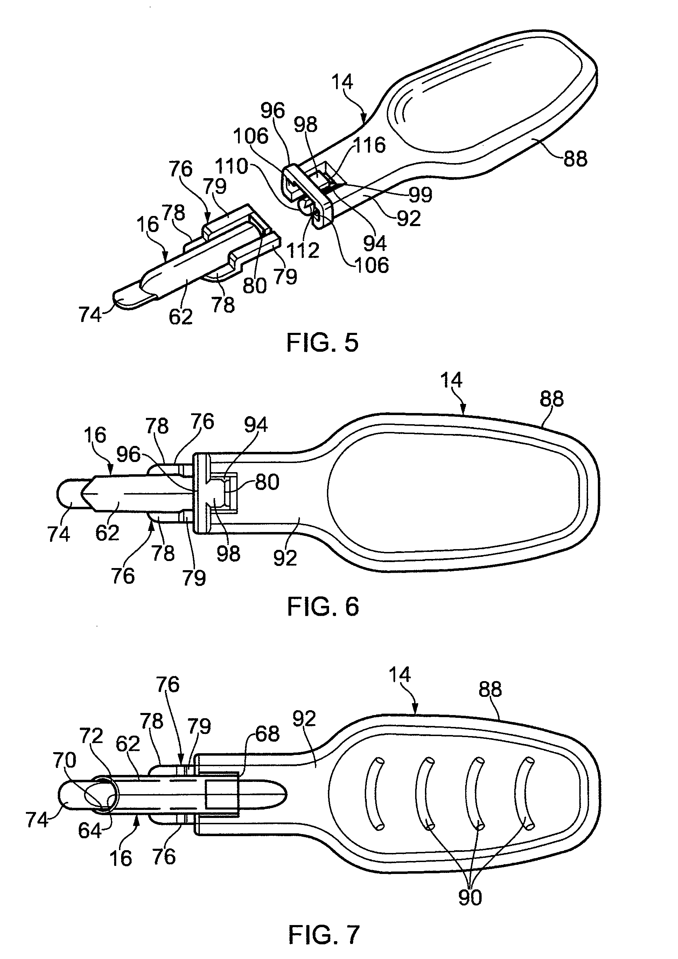

As shown best in Figures 5, 6, 7, 9, 10, and 11, the cartridge 16 will normally be moulded from a transparent to translucent plastic material. The sidewall forming its tubular body 62 defines a longitudinal bore or chamber 64, preferably of generally elliptical cross section, extending between an open posterior end 66 and open anterior end 70 surrounded, respectively, by a generally annular end surface 68 and a bevelled end surface 72.

A glide member 74 (in the form of a blade or tongue-like element) extends forwardly from the anterior end 70 of the sidewall of the body 62 and is of generally planar form (oriented, in a crosswise direction, parallel to the major elliptical axis of the chamber 64). Stepped flanges, generally designated by the numeral 76, are formed along the opposite sides of the cartridge body 62, each flange 76 consisting of an anterior element 78 and a posterior element 79, the end faces of which posterior elements 79 are coplanar with the generally annular surface 68 surrounding the posterior end opening 66. A catch element 80 is formed at the posterior end of the cartridge 16, between the flange elements 79, and provides means for disengageably securing the cartridge 16 to the handle 14 when the components are properly assembled with one another, as will be described presently.

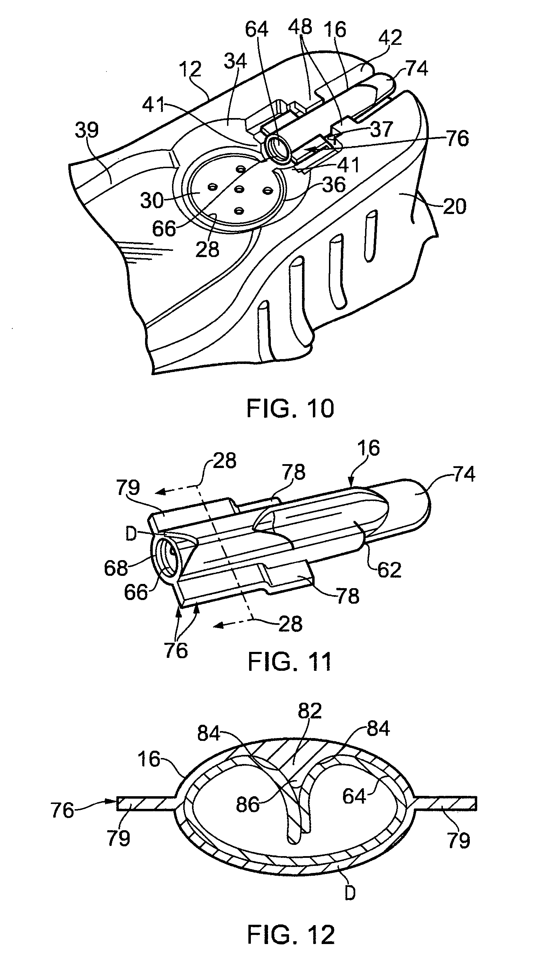

As best seen in Figure 12, an optional elongate ridge or protrusion 82 extends longitudinally along most of the length of the body 62 of the cartridge 16 and projects into the bore or chamber 64. The ridge 82 has convexly curved lateral bearing surfaces 84, which extend lengthwise along its opposite sides and terminate in a common, rounded apex 86.

The ridge or protrusion 82, where provided, is complementary to the ridge or protrusion 108 in the funnel structure 105 of the insert member 100 when the insert member 100 and the cartridge 16 are properly assembled onto the preparation base 12 as shown in Figure 1.

As shown best in Figures 5, 6 and 7, the handle component 14 of the system includes a relatively broad posterior portion 88 on which is formed (on the side that faces upwardly in the orientation of cornea implantation) raised rib elements 90, and a narrower anterior portion 92 into which is formed an axially extending indentation 94. A collar element 96 partially surrounds the end of the anterior portion 92, and is formed with a small prong 98 having an underlying tab element 99 across an inner edge. A pair of parallel slots 106 extend inwardly along the opposite sides of indentation 94, and serve to slidably receive the posterior flange elements 79 on the cartridge body 62; it will be appreciated that the cartridge 16 can be assembled with the handle 14 in only one orientation of rotation about its longitudinal axis.

When the cartridge 16 is fully engaged with the handle 14, a finger 110 is disposed within the bore 64 of the cartridge 16, with an apical portion 86 of the ridge 82 seated in the groove 112 along the finger 110. A first wall portion near the inner end of the slot bears upon the proximate end of the ridge 82, and a second wall portion 116 bears upon the confronting portion of the annular surface 68 at the posterior end of the cartridge bore 64. This structure produces an effective liquid-tight seal at the posterior end of the cartridge, which is held in assembly therewith due to the snap-fit engagement of the tab 99 on the prong 98 of the handle collar 96 in front of the catch 80 on the cartridge 16 (the tab having a bevel to facilitate assembly, and the prong 98 being sufficiently resiliently deflectable to enable disassembly, as appropriate).

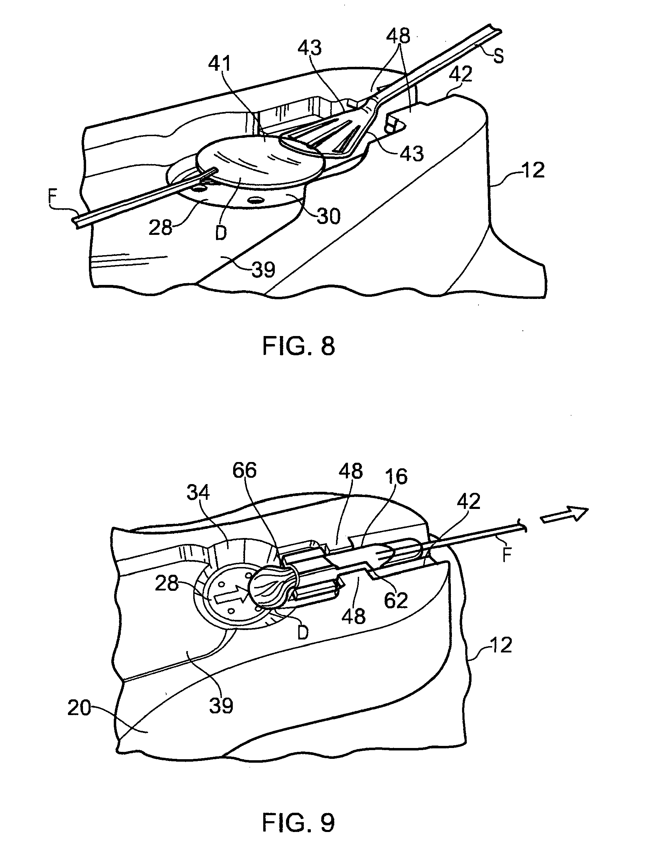

Use of the system of Figures 1 through 7 will be evident from the foregoing description, particularly taken with reference to Figures 8 through 15 (where the insert member 100 is omitted to allow a better view). As depicted in Figure 8, an implant "D" is initially placed into the well 28, with the endothelial surface facing upwardly, using a Paton's spatula "S" and forceps "F". A cartridge 16 is then assembled on the pedestal portion 20 of the preparation base 12, with the anterior flange elements 78 sildably engaged within the grooves 43 that are formed under the lugs 48. It will be appreciated that this structure permits the cartridge to be received in only a single orientation of rotation about its longitudinal axis, and by pushing it forwardly from a position over the well 28; removal requires movement of the cartridge, relative to the base, in the opposite direction.

The implant "D" is then drawn through the posterior end opening 66 of the body 62 of the cartridge 16 and into its bore 64, using forceps "F" introduced through the anterior opening 70 to grip the stromal edge and apply a pulling force thereupon, as indicated by the open arrows in Figure 10. In the course of drawing the implant "D" into the bore 64, its lateral edges are curled inwardly, by contact with the inner surface of the funnel structure 105 and with the bearing surfaces 84 of the ridge 82 (albeit either feature may suffice), ultimately to assume a double coil configuration, as shown in Figures 11 and 12, wherein there is seen to be no contact between any epithelial areas of the implant; it is also seen Figure 12 that the implant may be asymmetric in its double coil configuration.

When the implant "D' is drawn fully into the chamber 64 (as depicted in Figure 11), the handle 14 is attached to the cartridge 16, with the finger 110 inserted into the bore 64 and the mating proximate surface elements in mutual contact, so as to effectively seal the bore, and with the tab 99 of the prong 98 engaged behind the catch element 80 so as to secure the assembly. It will be appreciated that, as depicted in for example Figures 1 and 2, the handle cartridge assembly is inverted relative to the orientation in which the implant "D" will be introduced for implantation.

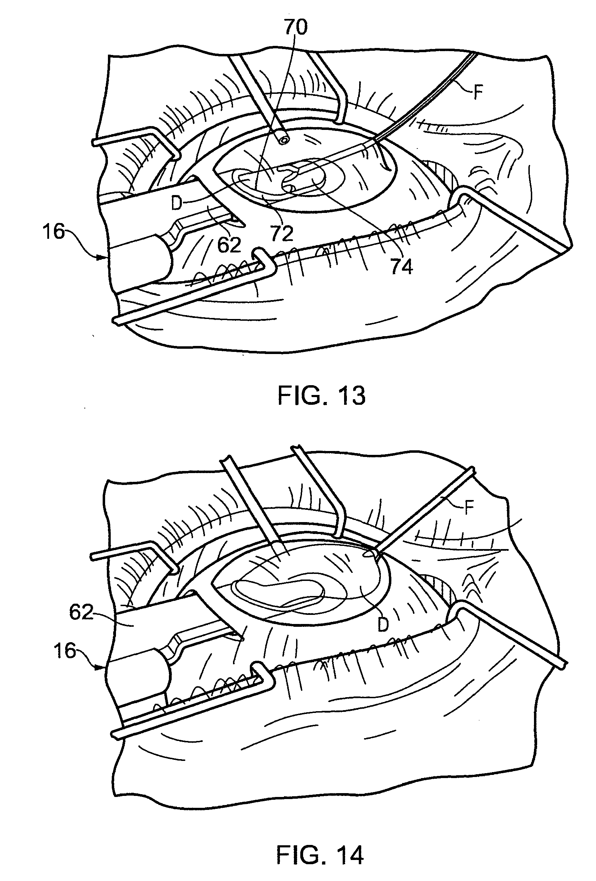

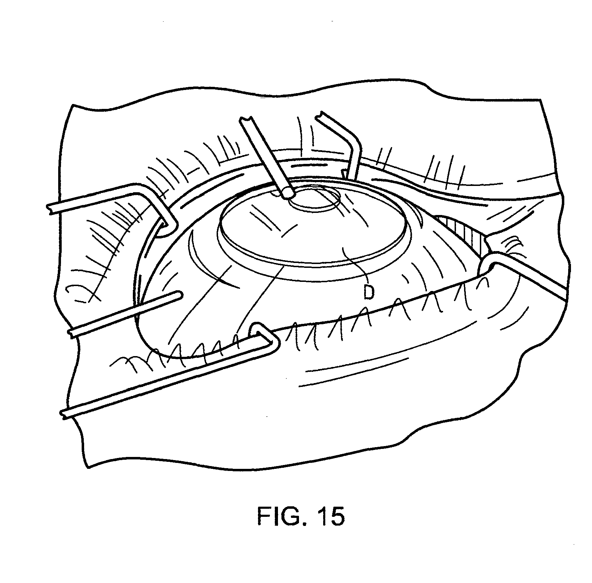

After the implant "D" is loaded into the cartridge, the assembly is removed from the preparation base 12 (by a rearwardly sliding action), turned right-side up and, in the initial phase of implantation, the blade element 74 and adjacent cartridge body portion are introduced through the scleral tunnel of the anterior chamber so as to form a complete seal of the wound; this phase is depicted in Figure 13. Subsequently, the implant "D" is pulled from the bore 64 through the anterior end opening 70 of the cartridge body 62 and into the anterior eye chamber (as also depicted in Figure 13), again using forceps "F" introduced through a diametrically located opening (nasal paracentesis). The implant "D" then automatically uncoils (albeit some manipulation using the forceps may be necessary or desirable); this phase of the process is depicted in Figure 14. Finally, a small air bubble is injected beneath the implant so as to prevent its decent onto the iris, the implant is released, and the cartridge and forceps are withdrawn; this phase is depicted in Figure 15.

Thus, it can be see that embodiments of the present invention provides an apparatus and method for preparing a corneal implant for implantation into the eye of a recipient without inducing significant endothelial damage. In accordance with the apparatus and method described, the implant is only temporarily deformed, for effective insertion, while protection against significant endothelial damage is afforded. It might be pointed out that, in its double coil configuration, corresponding opposite lateral portions of the implant need not be in mutual registration; useful results may be achieved when, for example, the implant is deformed to a 60:40 ratio of asymmetry in the double coil configuration.

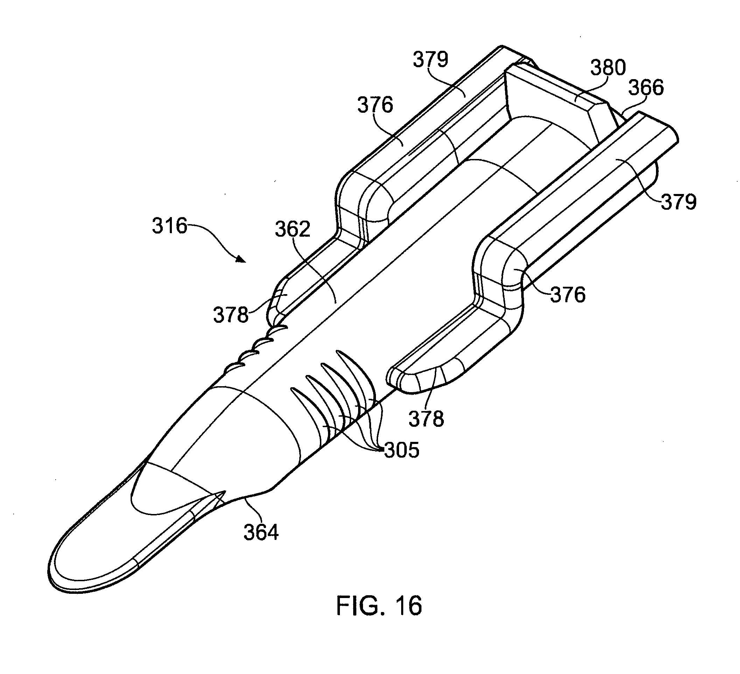

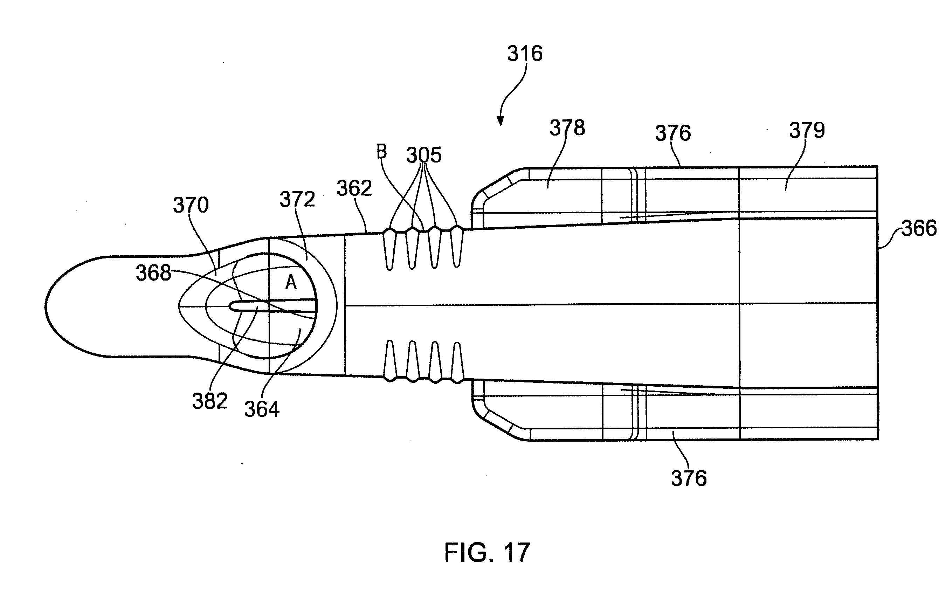

Figures 16 and 17 show an alternative cartridge 316 that is similar in construction to the cartridge 16 of Figure 11. The sidewall forming the tubular body 362 defines a longitudinal bore or chamber 364, preferably of generally elliptical cross section, extending between an open posterior end 366 and open anterior end 370 surrounded, respectively, by a generally annular end surface 368 and a bevelled end surface 372.

A glide member 374 (in the form of a blade or tongue-like element) extends forwardly from the anterior end 370 of the sidewall of the body 362 and is of generally planar form (oriented, in a crosswise direction, parallel to the major elliptical axis of the chamber 364). Stepped flanges, generally designated by the numeral 376, are formed along the opposite sides of the cartridge body 362, each flange 376 consisting of an anterior element 378 and a posterior element 379. A catch element 380 is formed at the posterior end of the cartridge 316, between the flange elements 379, and provides means for disengageably securing the cartridge 316 to the handle 14 when the components are properly assembled with one another.

As best seen in Figure 17, an optional elongate ridge or protrusion 382 extends longitudinally along most of the length of the body 362 of the cartridge 316 and projects into the bore or chamber 364. The ridge 382 has convexly curved lateral bearing surfaces 384, which extend lengthwise along its opposite sides and terminate in a common, rounded apex 386.

The ridge or protrusion 382, where provided, is complementary to the ridge or protrusion 108 in the funnel structure 105 of the insert member 100 when the insert member 100 and the cartridge 316 are properly assembled onto the preparation base 12 as shown in Figure 1.

Of particular note with respect to the cartridge 316 is that the tubular body 362 tapers inwardly from the rearward end 366 to the forward end 370, at least along a portion thereof. The diameter or cross-section of the longitudinal bore or chamber 364 is greater at the rearward end 366 than at for the forward end 370, and the width of the glide member 374 may therefore be smaller than the glide member 74 of the Figure 11 embodiment.

In a current embodiment suited for implantation of a donor cornea implant of diameter 9mm, this may be double coiled and pulled through a minimum bore diameter of 2.57mm at the forward end 370 without endothelial touch or overlap in embodiments with a ridge or protrusion 382. In embodiments without a ridge or protrusion 382, the implant may be coiled through a minimum bore diameter of 2.9mm at the forward end 370 without endothelial touch or overlap. As such, the minimum bore diameter in certain embodiments may be in the region of 2.60 to 3.00mm. Accounting for the thickness of the sidewall, the tubular body 362 may be tapered such that the width of the forward end 370 is approximately 2.7 to 3.0mm, while the width of the rearward end 366 is approximately 3.5 to 4.0mm.

In addition, one or more generally parallel ridges, ribs or grooves 305 are provided on the outer surface of the tubular body 362 so as to help resist against extrusion of the cartridge 316 from the anterior chamber of an eye when inserted therein through an incision as shown, for example, in Figures 13 and 14.

## Claims

1. A system for use in preparing a lenticular or corneal implant for delivery to an operating site and insertion into a recipient's eye, the system comprising:
a cartridge (16) comprising a generally tubular portion (62) including a sidewall defining a longitudinal bore (64), of curvilinear cross section, having open forward (70) and rearward ends (66);
a preparation base (12) comprised of a lower portion (18) and an upper portion (20), said upper portion having a recess 200 and having cartridge-receiving structure for removably engaging said cartridge in position with said open rearward end of said longitudinal bore thereof proximate one side of said recess, and for allowing facile removal of said cartridge from said base; and
an insert member (100) having structure for removably engaging said insert member in said recess, said insert member having a portion defining a platform 101 for receiving the implant, and a portion adjacent the platform defining funnel structure (105) having a wider end (107) facing the platform, and a narrower end (106) facing the open rearward end of said cartridge when the system is assembled; and
wherein the insert member has a saddle-shaped configuration, adapted to straddle and snugly engage the recess in the upper portion of the preparation base.

2. A system as claimed in claim 1, wherein the cartridge comprises attaching structure (80) adjacent a rearward end of said tubular portion for disengageable attachment to a handle (14).

3. A system as claimed in claim 2, further comprising a handle (14) having a gripping portion (88, 90) and means (94) adjacent one end for disengageable attachment to said attaching structure of said cartridge, said handle, when attached, enabling facile manipulation of said cartridge.

4. A system as claimed in any preceding claim, wherein at least one of said cartridge and said funnel structure has structure (82, 108) for inducing inward curling of opposite lateral edges of the implant in the course of passing from said platform to said forward end of said longitudinal bore of said cartridge.

5. A system as claimed in any preceding claim, wherein the narrower end of the funnel structure of the insert defines an aperture similar in cross-sectional area to the cross section of the longitudinal bore or the cartridge.

6. A system as claimed in any preceding claim, wherein the funnel structure includes a ridge element (108) extending longitudinally along at least a portion of funnel structure proximate the narrower end thereof and projecting inwardly thereinto and serving to induce curling of lateral edges of the implant during longitudinal movement therealong.

7. A system as claimed in any preceding claim, wherein the longitudinal bore of the cartridge includes a ridge element (82) extending longitudinally along at least a portion of the bore and projecting inwardly thereinto from the sidewall and serving to induce curling of lateral edges of the implant during longitudinal movement therealong.

8. A system as claimed in any preceding claim, wherein the cartridge has a blade portion (74) extending forwardly from said sidewall beyond said forward open end of said bore.

9. A method of preparing a lenticular or corneal implant prior to surgery, using the system of any one of claims 1 to 8, comprising the steps:
(1) supporting the implant in position adjacent the open rearward end of said longitudinal bore of said tubular portion of said cartridge; and
(2) drawing said implant into said bore, to reside therein, by force applied from said open forward end of said bore.

10. A method according to claim 9, wherein the cartridge includes attaching structure adjacent a rearward end of said tubular portion for disengageable attachment to a handle, and comprising the further steps:
(3) providing a handle having a gripping portion and means adjacent one end for disengageable attachment to said attaching structure of said cartridge; and
(4) attaching said handle to said cartridge using said means for disengageable attachment of said handle and said attaching structure of said cartridge.

11. A method according to claim 9 or 10, wherein the implant is an endothelial corneal implant, and is supported adjacent the open rearward end of the longitudinal bore with its endothelial side uppermost.

12. A method according to claim 11, including the further step of causing said implant to assume a double coil configuration, with no endothelial surface areas in mutual contact with one another, in the course of drawing said donor cornea into and through said bore of said cartridge.

## Patentansprüche

1. System zur Verwendung bei der Vorbereitung eines Linsen- oder Homhautimplantats zur Abgabe an einen Operationsort und zum Einsetzen in das Auge eines Empfängers, wobei das System Folgendes umfasst:
eine Kartusche (16), die einen im Allgemeinen röhrenförmigen Abschnitt (62) umfasst, der eine Seitenwand umfasst, die eine Längsbohrung (64) mit krummlinigem Querschnitt definiert und offene vordere (70) und hintere Enden (66) umfasst;
eine Vorbereitungsbasis (12), die aus einem unteren Abschnitt (18) und einem oberen Abschnitt (20) besteht, wobei der obere Abschnitt eine Vertiefung (200) aufweist und eine Kartuschenaufnahmestruktur aufweist, um die Kartusche abnehmbar in die richtige Position mit dem offeneren hinteren Ende ihrer Längsbohrung in der Nähe einer Seite der Vertiefung einzurücken und um ein müheloses Abnehmen der Kartusche von der Basis zu ermöglichen; und
ein Einlageelement (100), das eine Struktur zum abnehmbaren Einrücken des Einlageelements in die Vertiefung aufweist, wobei das Einlageelement einen Abschnitt, der eine Plattform (101) definiert, um das Implantat aufzunehmen, und einen Abschnitt neben der Plattform aufweist, der eine Trichterstruktur (105) definiert, die ein breiteres Ende (107) gegenüber der Plattform und ein schmaleres Ende (106) gegenüber dem offenen hinteren Ende der Kartusche aufweist, wenn das System zusammengebaut ist; und
wobei das Einlageelement eine sattelförmige Konfiguration aufweist, die geeignet ist, um auf der Vertiefung in dem oberen Abschnitt der Vorbereitungsbasis zu sitzen und fest in diese einzurücken.

2. System nach Anspruch 1, wobei die Kartusche eine Anbringungsstruktur (80) neben einem hinteren Ende des röhrenförmigen Abschnitts zur ausrückbaren Anbringung an einem Griff (14) umfasst.

3. System nach Anspruch 2, ferner umfassend einen Griff (14), der einen Greifabschnitt (88, 90) und neben einem Ende Mittel (94) zur ausrückbaren Anbringung an der Anbringungsstruktur der Kartusche aufweist, wobei der Griff, wenn er angebracht ist, eine mühelose Handhabung der Kartusche ermöglicht.

4. System nach einem der vorhergehenden Ansprüche, wobei mindestens eine von der Kartusche und der Trichterstruktur eine Struktur (82, 108) aufweist, um ein nach innen Einrollen der gegenüberliegenden seitlichen Ränder des Implantats beim Übergang von der Plattform zu dem vorderen Ende der Längsbohrung der Kartusche herbeizuführen.

5. System nach einem der vorhergehenden Ansprüche, wobei das schmalere Ende der Trichterstruktur der Einlage eine Öffnung definiert, deren Querschnittsfläche ähnlich wie die Querschnittsfläche der Längsbohrung oder der Kartusche ist.

6. System nach einem der vorhergehenden Ansprüche, wobei die Trichterstruktur ein Rippenelement (108) umfasst, das sich längsseitig entlang mindestens einem Abschnitt der Trichterstruktur in der Nähe ihres schmaleren Endes erstreckt und darin nach innen vorspringt und dazu dient, ein Einrollen der seitlichen Ränder des Implantats während einer Längsbewegung daran entlang herbeizuführen.

7. System nach einem der vorhergehenden Ansprüche, wobei die Längsbohrung der Kartusche ein Rippenelement (82) umfasst, das sich längsseitig entlang mindestens einem Abschnitt der Bohrung erstreckt und darin von der Seitenwand aus nach innen vorsteht und dazu dient, ein Einrollen der seitlichen Ränder des Implantats während einer Längsbewegung daran entlang herbeizuführen.

8. System nach einem der vorhergehenden Ansprüche, wobei die Kartusche einen Schneidenabschnitt (74) aufweist, der sich von der Seitenwand aus nach vorne über das vordere offene Ende der Bohrung hinaus erstreckt.

9. Verfahren zum Vorbereiten eines Linsen- oder Hornhautimplantats vor einer Operation unter Verwendung des Systems nach einem der Ansprüche 1 bis 8, umfassend folgende Schritte:
(1) Halten des Implantats in der richtigen Position neben dem offenen hinteren Ende der Längsbohrung des röhrenförmigen Abschnitts der Kartusche; und
(2) Ziehen des Implantats in die Bohrung, um darin zu liegen, durch eine Kraft, die von dem offenen vorderen Ende der Bohrung aus ausgeübt wird.

10. Verfahren nach Anspruch 9, wobei die Kartusche eine Anbringungsstruktur neben einem hinteren Ende des röhrenförmigen Abschnitts zur ausrückbaren Anbringung an einem Griff umfasst, und umfassend die folgenden weiteren Schritte:
(3) Bereitstellen eines Griffs mit einem Greifabschnitt und Mitteln neben einem Ende zur ausrückbaren Anbringung an der Anbringungsstruktur der Kartusche; und
(4) Anbringen des Griffs an der Kartusche unter Verwendung der Mittel zur ausrückbaren Anbringung des Griffs und der Anbringungsstruktur der Kartusche.

11. Verfahren nach Anspruch 9 oder 10, wobei das Implantat ein endotheliales Hornhautimplantat ist und neben dem offenen hinteren Ende der Längsbohrung mit seiner endothelialen Seite ganz oben gehalten wird.

12. Verfahren nach Anspruch 11, umfassend den weiteren Schritt des Verursachens, dass das Implantat eine Doppelspiralenkonfiguration annimmt, wobei sich keine endothelialen Mantelflächen in gegenseitigem Kontakt miteinander befinden während die Spenderhornhaut in und durch die Bohrung der Kartusche gezogen wird.

## Revendications

1. Système destiné à une utilisation dans la préparation d'un implant cornéen ou lenticulaire à poser sur un site d'exploitation et l'insertion dans l'oeil d'un receveur, le système comprenant :
une cartouche (16) comprenant une partie généralement tubulaire (62) incluant une paroi latérale définissant un alésage longitudinal (64), de section transversale curviligne, ayant des extrémités avant (70) et arrière (66) ouvertes ;
une base de préparation (12) constituée d'une partie inférieure (18) et d'une partie supérieure (20), ladite partie supérieure ayant un creux (200) et une structure de réception de cartouche pour engager ladite cartouche de manière amovible dans ladite extrémité arrière ouverte de son dit alésage longitudinal à proximité d'un côté dudit creux, et pour permettre le retrait facile de ladite cartouche de ladite base ; et
un élément d'insertion (100) ayant une structure pour engager de manière amovible ledit élément d'insertion dans ledit creux, ledit élément d'insertion ayant une partie définissant une plate-forme (101) pour recevoir l'implant, et une partie adjacente à la plateforme définissant une structure en forme d'entonnoir (105) ayant une extrémité plus large (107) faisant face à la plate-forme, et une extrémité plus petite (106) faisant face à l'extrémité arrière ouverte de ladite cartouche lorsque le système est monté ; et
dans lequel l'élément d'insertion a une configuration en forme de selle, adaptée pour mettre à cheval et parfaitement engager le creux dans la partie supérieure de la base de préparation.

2. Système tel que revendiqué dans la revendication 1, dans lequel la cartouche comprend une structure de fixation (80) adjacente à une extrémité arrière de ladite partie tubulaire pour la fixation débrayable à un manche (14).

3. Système tel que revendiqué dans la revendication 2, comprenant en outre un manche (14) ayant une partie de préhension (88, 90) et des moyens (94) adjacents à une extrémité pour la fixation débrayable à ladite structure de fixation de ladite cartouche, ledit manche, lorsqu'il est attaché, permettant une manipulation facile de ladite cartouche.

4. Système tel que revendiqué dans l'une quelconque des revendications précédentes, dans lequel au moins un élément entre ladite cartouche et ladite structure en forme d'entonnoir a une structure (82, 108) pour induire une ondulation vers l'intérieur des bords latéraux opposés de l'implant au cours du passage de ladite plate-forme vers ladite extrémité avant dudit alésage longitudinal de ladite cartouche.

5. Système tel que revendiqué dans l'une quelconque des revendications précédentes, dans lequel l'extrémité la plus petite de la structure en forme d'entonnoir de l'insertion définit une ouverture similaire dans la zone de section transversale à la section transversale de l'alésage longitudinal ou de la cartouche.

6. Système tel que revendiqué dans l'une des revendications précédentes, dans lequel la structure en forme d'entonnoir inclut un élément de faîtage (108) s'étendant longitudinalement le long d'au moins une partie de la structure en forme d'entonnoir à proximité de son extrémité la plus étroite, faisant saillie vers l'intérieur de celle-ci et servant à induire une ondulation des bords latéraux de l'implant durant le mouvement longitudinal sur sa longueur.

7. Système tel que revendiqué dans une des revendications précédentes, dans lequel l'alésage longitudinal de la cartouche inclut un élément de faîtage (82) s'étendant longitudinalement le long d'au moins une partie de l'alésage et faisant saillie vers l'intérieur de celle-ci à partir de la paroi latérale et servant à induire une ondulation des bords latéraux de l'implant durant le mouvement longitudinal sur sa longueur.

8. Système tel que revendiqué dans l'une quelconque des revendications précédentes, dans lequel la cartouche a une partie en lame (74) s'étendant vers l'avant à partir de ladite paroi latérale au-dessus de ladite extrémité ouverte à l'avant dudit alésage.

9. Procédé de préparation d'un implant cornéen ou lenticulaire avant l'opération chirurgicale, utilisant le système de l'une quelconque des revendications 1 à 8 comprenant les étapes :
(1) de support de l'implant en position adjacente à l'extrémité arrière ouverte dudit alésage longitudinal de ladite partie tubulaire de ladite cartouche ; et
(2) de tirage dudit implant dans ledit alésage, pour y résider, par la force appliquée à partir de ladite extrémité arrière ouverte dudit alésage.

10. Procédé selon la revendication 9, dans lequel la cartouche inclut la fixation de la structure adjacente à une extrémité arrière de ladite partie tubulaire pour la fixation amovible à un manche, et comprenant les étapes supplémentaires :
(3) de fourniture d'un manche ayant une partie de préhension et des moyens adjacents à une extrémité pour une fixation amovible à ladite structure de fixation de ladite cartouche ; et
(4) de fixation dudit manche à ladite cartouche en utilisant les dits moyens pour la fixation amovible dudit manche et de ladite structure de fixation de ladite cartouche.

11. Procédé selon la revendication 9 ou 10, dans lequel l'implant est un implant cornéen endothélial, et est supporté de manière adjacente à l'extrémité arrière ouverte de l'alésage longitudinal avec le côté endothélial étant le plus élevé.

12. Procédé selon la revendication 11, incluant l'étape supplémentaire consistant à inciter ledit implant à prendre une configuration à double bobine, aucune des surfaces endothéliales n'étant en contact mutuel avec l'autre, au cours du tirage de ladite cornée du donneur dans et à travers ledit alésage de ladite cartouche.
